# EUROPEAN PATENT SPECIFICATION

(11) **EP 4 547 732 B1**
(45) Date of publication and mention of the grant of the patent: **06.05.2026**
(21) Application number: 23734640.8
(22) Date of filing: 22.06.2023
(51) Int. Cl.: C08G 63/688, C08G 63/685, A61Q 5/04

(54) **POLYESTER POLYMER FOR USE IN HAIR CARE**
POLYESTERPOLYMER ZUR VERWENDUNG IN DER HAARPFLEGE
POLYMÈRE DE POLYESTER DESTINÉ À ÊTRE UTILISÉ DANS LE SOIN DES CHEVEUX

(30) Priority: 28.06.2022 IN 202221037108; 26.08.2022 EP 22192291
(43) Date of publication of application: 07.05.2025
(73) Proprietor: SYENSQO SA, 1130 Brussels (BE)
(72) Inventor: CHANDA, Sananda, Vadodara, Gujarat 391 770 (IN); SHIVANANDAREDDY, Avinash, Vadodara, Gujarat 391 770 (IN); MAKWANA, Ketan, BHARUCH, 394116 (IN); CHAKRABORTY, Kaustav, BHARUCH, 394116 (IN); WILSON, David, James, 93300 Aubervilliers (FR); MASTROIANNI, Sergio, 69190 Saint-Fons (FR)
(74) Representative: Kraus & Lederer PartGmbB
(86) International application number: PCT/EP2023/066991
(87) International publication number: WO 2024/002862

(56) References cited:
- US-B1- 6 749 836

## Description

### FIELD OF THE INVENTION

The present invention provides a polyester polymer for use in hair care formulations, namely in hair straightening compositions.

### BACKGROUND OF THE INVENTION

Human hair is made of keratin which in turn is made of polypeptide chains bonded together by disulfide bonds, hydrogen bonds and salt linkages. Temporary straightening, using physicochemical techniques such as dryer, flat iron and the old hot comb, lasts only until the next wash. More permanent straightening of hair can be achieved by chemically altering the disulfide bonds of keratin, but this weakens the hair fibers. Conditioning polymers can also improve the hair manageability and make the hair softer and smoother. Cationic polymers are preferred as they are hold by the negatively charged hair proteins by electrostatic forces, whereas nonionic polymers are more easily washed off by surfactants. However, most of the currently available conditioning polymers are not biodegradable so when they end up in wastewater, they potentially lead to the formation of microplastics.

US 5662893 discloses an hair spray comprising a sulfopolyester which contains repeat units from 20 to 26 mole percent dimethyl-5-sodiosulfoisophthalate and 74 to 80 mole percent isophthalic acid, based on 100 mole percent dicarboxylic acid; 10 to 30 mole percent 1,4-cyclohexanedimethanol and 70 to 90 mole percent diethylene glycol, based on 100 mole percent diol. This document does not address the problem of long term hair straightening, but merely addresses the problem of providing human hair with a particular shape or configuration by applying a thin film of a resin or gum onto the hair to adhere adjacent hairs together so that they retain the particular shape or configuration at the time of application. It doesn't address the problem of degradability either. US 6 749 836 B1 discloses a hair care formulation comprising a sulfopolyester comprising the condensation polymerization product of *inter alia* sodiosulfoisophthalate, isophthalic acid and cyclohexanedimethanol.

An object of the present invention is to provide a polyester polymer that is effective in long-term hair straightening. Another object of the present invention is to provide a polyester polymer that is biodegradable.

In another aspect of the present invention, a method of synthesis for a polyester polymer is provided.

Finally, the present invention also relates to the use of given polyester polymers in hair care compositions, especially in hair straightening compositions.

### DETAILED DESCRIPTION OF THE INVENTION

The polyester polymer of the present invention is prepared by polycondensation of at least the following monomers:
(a) an aliphatic/aromatic unsulfonated dicarboxylic acid/ester;
(b) an aliphatic diol;
(c) an aliphatic/aromatic sulfonated dicarboxylic acid/ester;
(d) an aliphatic/aromatic secondary or tertiary amine bearing two carboxylic acid/ester functions, or an aliphatic/aromatic quaternized amine bearing two carboxylic acid/ester functions.

The aliphatic unsulfonated dicarboxylic acid/ester according to the invention may be linear or cyclic. Preferred linear aliphatic unsulfonated dicarboxylic acids/esters according to the invention are alkyldicarboxylic acids/esters of formula ROOC-(CH2)n-COOR (I) where n = 2-4 and R is H, a C1 to C8 alkyl or phenyl group. Preferred cycloaliphatic unsulfonated dicarboxylic acids/esters according to the invention are based on cycles with 5 or 6 C atoms i.e. cyclopentanedicarboxylic acids/alkyl esters or cyclohexanedicarboxylic acids/alkyl esters, in particular 1,2-cyclopentanedicarboxylic acid, 1,3-cyclopentanedicarboxylic acid, 1,3-cyclohexanedicarboxylic acid or 1,4-cyclohexanedicarboxylic acid and their corresponding alkyl esters where the alkyl group can vary from methyl to octyl or can be a phenyl group.

Preferred aromatic unsulfonated dicarboxylic acids/esters according to the invention are terephthalic acid/alkyl esters and isophthalic acid/alkyl esters where the alkyl group can vary from methyl to octyl or can be a phenyl group.

The aliphatic/aromatic unsulfonated dicarboxylic acid/ester (a) of the invention is preferably an aliphatic one, more preferably a cyclohexane derivative. In particular 1,4-cyclohexanedicarboxylic acid (CHDA) gives good results in the frame of the present invention.

The aliphatic diol used in the present invention can be a linear aliphatic diol selected from the group consisting of alkyl diols like ethylene glycol or propylene glycol, diethylene glycol, triethylene glycol or a polyethylene glycol having an ethylene oxide number ranging from 4 to 75. Alternatively, it can be a cyclic saturated diol preferably comprising 5 or 6 C atoms i.e. a cyclopentane diol or a cyclohexane diol, in particular 1,2-cyclopentane diol, 1,3-cyclopentane diol, 1,3-cyclohexane diol or 1,4-cyclohexane diol.

Preferably, linear aliphatic diols are used, more preferably alkyl diols, in particular ethylene glycol (EG).

The aliphatic/aromatic sulfonated dicarboxylic acid/ester (c) has at least one sulfonic acid group, preferably in the form of an alkali metal (preferably sodium) sulfonate, and two acid/ester functional groups attached to one or a number of aromatic rings, when aromatic dicarboxylic acids or their alkyl diesters are involved, or to the aliphatic chain when aliphatic dicarboxylic acids/alkyl diesters are involved, where the alkyl group can vary from methyl to octyl or can be a phenyl group .

Aromatic sulfonated dicarboxylic acids/esters monomers that can be used in the frame of the invention are preferably isophthalic acid/esters, terephthalic acid/esters and naphthalenedicarboxylic acids/esters. Preferred ones are 2-sodiosulfoisophthalic acid/ester, 4-sodiosulfoisophthalic acid/ester, 5-sodiosulfoisophthalic acid/ester, 2-sodiosulfoterephthalic acid/ester, 2,6-dicarboxyl naphthalene-4-sodiosulfonic acid/ester and 2,6-dicarboxyl naphthalene-7-sodiosulfonic acid/ester. Aliphatic sulfonated dicarboxylic acids/esters that can be used in the frame of the present invention are dialkyl sodium sulfosuccinates.

Aromatic sulfonated dicarboxylic acid/ester monomers are preferably used in the frame of the invention, more preferably 5-sodiosulfoisophthalic acid/esters, in particular 5-sodiosulfoisophthalic acid (SSIA).

As examples of aliphatic secondary or tertiary amines according to the invention, mention can be made of N-methyldiethanolamine, ethylenediamine N,N' diacetic acid/alkyl diesters and iminodiacetic acid/ alkyl diesters, where the alkyl group can vary from methyl to octyl or can be a phenyl group.

As examples of aromatic secondary or tertiary amines according to the invention, mention can be made of pyridine derivatives like pyridine-2,6-dicarboxylic acid/alkyl diesters, pyridine-2,4-dicarboxylic acid/alkyl diesters, pyridine-3,5-dicarboxylic acid/alkyl diesters, pyridine-2,3-dicarboxylic acid/alkyl diesters and pyridine-2,5-dicarboxylic acid/alkyl diesters; of pyrazine derivatives like pyrazine-2,6-dicarboxylic acid/alkyl diesters, pyrazine-2,3-dicarboxylic acid/alkyl diesters and pyrazine-2,5-dicarboxylic acid/alkyl diesters; of imidazole derivatives like 1H-imidazole-2,5-dicarboxylic acid/alkyl diesters and 1H-imidazole-4,5-dicarboxylic acid/alkyl diesters; and of indole derivatives like 1H-indole-4,6-dicarboxylic acid/alkyl diesters, 1H-indole-2,5-dicarboxylic acid/alkyl diesters, 1H-indole-2,6-dicarboxylic acid/alkyl diesters, 1H-indole-3,5-dicarboxylic acid/alkyl diesters and 1H-indole-2,3-dicarboxylic acid/alkyl diesters, where the alkyl group can vary from methyl to octyl or can be a phenyl group.

As examples of aliphatic quaternized amines according to the invention, mention can be made of the quaternized forms of the above mentioned aliphatic tertiary amines, in particular of the quaternized forms of N-methyldiethanolamine.

As examples of aromatic quaternized amines according to the invention, mention can be made of the quaternized forms of the above mentioned aromatic tertiary amines, in particular of the quaternized forms of pyridine-2,6-dicarboxylic acid/esters, like for instance 2,6-bis(methoxycarbonyl)-1-methylpyridin-1-ium trifluoromethanesulfonate, chloride, bromide, iodide, sulfate.

The synthesis of 2,6-bis(methoxycarbonyl)-1-methylpyridin-1-ium trifluoromethanesulfonate has been described namely in the article "Photodetachment of Zwitterions: Probing Intramolecular Coulomb Repulsion and Attraction in the Gas Phase Using Pyridinium Dicarboxylate Anions", J. AM. CHEM. SOC. 2003, 125, 296-304.

Aromatic tertiary amines or quaternized amines are preferred, especially the above mentioned pyridine derivatives, more particularly pyridine-2,6-dicarboxylic acid (PDA) and 2,6-bis(methoxycarbonyl)-1-methylpyridin-1-ium trifluoromethanesulfonate (quatPDA).

In a preferred embodiment, the polyester polymer of the present invention is prepared by polycondensation of the following monomers:
(a) an aliphatic unsulfonated dicarboxylic acid/ester, preferably a cyclohexane derivative, in particular 1,4-cyclohexanedicarboxylic acid (CHDA);
(b) a linear aliphatic diol, in particular ethylene glycol (EG);
(c) an aromatic sulfonated dicarboxylic acid/ester, preferably 5-sodiosulfoisophthalic acid/ester, in particular 5-sodiosulfoisophthalic acid (SSIA);
(d) an aromatic tertiary amine or quaternized amine bearing two carboxylic acid/ester functions, preferably a pyridine derivative, in particular pyridine-2,6-dicarboxylic acid (PDA) or 2,6-bis(methoxycarbonyl)-1-methylpyridin-1-ium trifluoromethanesulfonate (quarPDA).

A particularly preferred polyester of the invention is prepared by polycondensation of the following monomers:
(a) 1,4-cyclohexanedicarboxylic acid (CHDA);
(b) ethylene glycol (EG);
(c) 5-sodiosulfoisophthalic acid (SSIA);
(d) pyridine-2,6-dicarboxylic acid (PDA) or 2,6-bis(methoxycarbonyl)-1-methylpyridin-1-ium trifluormethanesulfonate (quatPDA).

The present invention also concerns a novel and inventive polyester polymer comprising the following repeat units: CHDA-EG, SSIA-EG and (quat)PDA-EG (i.e. PDA-EG or a quatPDA-EG).

In a particularly preferred embodiment, the polymer is an aliphatic aromatic polyester which is derived from (a) a mixture of dicarboxylic acids comprising cyclohexane dicarboxylic acid (mole percent varied from 10 to 40 %), 5-sodio sulfoisophthalic acid (mole percent varied from 5 to 20 %) and 2,6 - pyridine dicarboxylic acid/ quaternized 2,6 - pyridine dicarboxylic acid (or ester) (mole percent varied from 5 to 20 %) and (b) a diol component, preferably ethylene glycol. The weight average molecular weight of the polyester can vary from 5000 to 15000 and the polyester is preferably soluble/dispersible in water.

Preferred polyesters according to the invention comprise at most 20mol% of aromatics (e.g. SSIA and (quat)PDA) in order to promote/facilitate biodegradability (see below for more details on biodegradability).

The present invention also concerns a process for synthesizing the above described polyester polymer by polycondensation of monomers (a), (b), (c) and (d), preferably in the presence of a catalyst. This catalyst is preferably a hydrolysis-stable catalyst, more preferably chosen from chelates of titanium salts or of zirconium salts derived from ethanol amines, separately and/or mixtures or solutions thereof. In particular, Titanium(IV) (triethanolaminato)isopropoxide gives good results. This compound is available as a 80wt% solution in isopropanol under the brand name Tyzor^{®} TE.

The polycondensation according to the invention is preferably initiated on the mixture of all monomers (a) to (d) i.e. monomers (a), (b), (c) and (d) are first mixed and then, reacted by polycondensation, preferably by raising temperature and/or reducing pressure. Alternatively, the polycondensation can be initiated on a mixture of only some of the monomers, the others being introduced in a delayed manner. Still another possibility is to prepare 2 or more prepolymers by polycondensation and then, to proceed to transesterification of the prepolymers.

In a preferred embodiment, the procedure for the preparation of the polyesters according to the invention is as follows. First, all the monomers are mixed in a reaction vessel and the mixture is heated from about 110 °C to 200 °C, preferably from 120 °C to 180 °C under nitrogen blanket. The reaction mixture is then preferably maintained at the same temperature for 30 to 240 minutes, preferably for 60 to 180 minutes under agitation. Subsequently, the reaction temperature is preferably raised to 200 °C and gradually a reduced pressure of 50 to 300 mbar, preferably of 100 to 200 mbar is achieved; under this condition ethylene glycol starts distilling and is preferably collected in a receiver. The reaction temperature is then preferably increased to between about 210 °C and 250 °C under reduced pressure. As the reaction achieves the desired temperature, the pressure is then preferably further reduced to about 10 mbar to 50 mbar, preferably to about 20 to 40 mbar. The reaction is then preferably maintained for 30 to 240 minutes, preferably for 60 to 180 minutes in this condition after which the polymer can be discharged in hot condition.

In one embodiment of the invention, the quaternized version of the polymer is obtained as a result of the quarternization of a tertiary amine group in the polyester backbone (post-polymerization modification). In this embodiment, the polyester is preferably dissolved in suitable solvent, mixed with a magnetic stirrer and heated to reflux. The quaternizing agent is then preferably added in a molar ratio tertiary amine (polyester) : quaternizing agent where the molar ratio is varied from 1:1.1 to 1:2, preferably from 1:1.2 to 1:1.8, with a syringe through a rubber septum. After the reaction, the solvent is removed and the resultant polyester is obtained.

The present invention also relates to the use of the above described polyester polymer in hair care compositions, in particular in hair straightening compositions.

### EXAMPLES

### Examples 1 to 7: Polyester polymers with PDA monomers : Synthesis & properties

A glass reactor equipped with an overhead stirrer, nitrogen inlet, condenser setup with receiving vessel and a solid addition port was charged with 228.71 (1.31 mol) of 1,4 CHDA, 46.4g (0.164 mol) of SSIA, 27.45g (0.164 mol) of PDA, 2.08g (6.57 mmol) of Titanium(IV) (triethanolaminato)isopropoxide solution(80 wt. % in isopropanol) (Tyzor^{®} TE) and 309.18g (4.93 mol) of EG. An excess of ethylene glycol (3 equivalent) was used to dissolve the SSIA; however, at the end of the reaction the excess ethylene glycol was distilled out (Refer to Table 1 for the initial and final feed of EG). Initially, the reaction mixture was heated to 160 °C under nitrogen blanket and the reaction mixture was maintained at the same temperature for 60 minutes under agitation, then the reaction temperature was raised to 200 °C and gradually a reduced pressure of 100 mBar was achieved, under this condition ethylene glycol started distilling and was collected in a receiver. The reaction temperature was then increased to 235 °C under reduced pressure. As the reaction achieved 235 °C the pressure was further reduced to 20 mBar. The reaction was maintained for 60 minutes in this condition after which the polymer was discharged in hot condition.

Characteristics of the polyester polymer obtained (Example 1): Molecular weight (Mw): 4600 Da (measured by gel permeation chromatography with hexafluoroisopropanol as eluent); Glass transition temperature (Tg): -3 °C
Following the protocol listed above, Examples 2 to 7 with different compositions were prepared, of which the characteristics/properties are listed in Table 1 below.

**Table 1**

| | CHDA | SSIA | PDA | EG (Initial feed) | EG (Final feed) | Tyzor TE | Mw (Da) | Tg |
|---|---|---|---|---|---|---|---|---|
| Ex. 2 | 2.89g (16.6 mmol) | 1.56g (5.54 mmol) | 0,92g (5.54 mmol) | 8.65g (138.65 mmol) | 1.72g (27.68 mmol) | 37.6mg (0.11 mmol) | Not done | 25 °C |
| Ex. 3 | 2.94g (16.9 mmol) | 1.27g (4.51 mmol) | 1.13g (6.76 mmol) | 8.835g (140.92 mmol) | 1.75g (28.17 mmol) | 35.7mg (0.113 mmol) | 8300 | 41 °C |
| Ex. 4 | 1.86g (10.7 mmol) | 2.26g (8.03 mmol) | 1.34g (8.03 mmol) | 8.39g (133.8 mmol) | 1.66g (26.76 mmol) | 33.9mg (0.107 mmol) | Not done | 71 °C |
| Ex. 5 | 1.90g (10.96 mmol) | 1.85g (6.57 mmol) | 1.64g (9.86 mmol) | 8.59g (137 mmol) | 1.7g (27.39mmol) | 34.7mg (0.11 mmol) | Not done | 63 °C |
| Ex. 6 | 1.82g (10.46 mmol) | 2.65g (9.41 mmol) | 1.04g (6.27 mmol) | 8.2g (130.8 mmol) | 1.62g (26.14 mmol) | 33.1mg (0.105 mmol) | Not done | 94 °C |
| Ex. 7 | 0.899g (5.17 mmol) | 2.92g (10.34 mmol) | 1.72g (10.34 mmol) | 8.11g (129.35 mmol) | 1.6g (25.85 mmol) | 32.7mg (0.103 mmol) | Not done | 102 °C |

### Examples 8 to 14: Polyester polymers with quatPDA monomers: Synthesis & properties

A glass reactor equipped with an overhead stirrer, nitrogen inlet, condenser setup with receiving vessel and a solid addition port was charged with 37.09 (0. 213 mol) of CHDA, 7.52g (0.026 mol) of SSIA, 9.59g (0.026 mol) of Pyridinium, 2,6-bis(methoxycarbonyl)-1-methylpyridin-1-ium trifluormethanesulfonate, 0.337g (1.067 mmol) of Titanium(IV) (triethanolaminato)isopropoxide solution (80 wt. % in isopropanol) (Tyzor^{®} TE) and 50.15g (0.808 mol) of Ethylene Glycol. An excess of ethylene glycol (3 equivalent) was used to dissolve the SSIA; however, at the end of the reaction the excess ethylene glycol was distilled out (Refer to Table 2 for the initial and final feed of EG). Initially, the reaction mixture was heated to 160 °C under nitrogen blanket and the reaction mixture was maintained at the same temperature for 60 minutes under agitation, then the reaction temperature was raised to 200 °C and gradually a reduced pressure of 100 mBar was achieved, under this condition ethylene glycol started distilling and was collected in a receiver. The reaction temperature was then increased to 235 °C under reduced pressure. As the reaction achieved 235 °C the pressure was further reduced to 20 mBar. The reaction was maintained for 60 minutes in this condition after which the polymer was discharged in hot condition.

Characteristics of the polyester polymer obtained (Example 8): Molecular weight (Mw): 29100 Da (measured by gel permeation chromatography with hexafluoroisopropanol as eluent); Glass transition temperature (Tg): -10 °C
Following the protocol listed above other examples with different compositions were prepared, of which the characteristics/properties are listed in Table 2 below.

**Table 2**

| | CHDA | SSIA | Q-PDA | EG (Initial feed) | EG (Final feed) | Tyzor | Mw (Da) | Tg |
|---|---|---|---|---|---|---|---|---|
| Ex. 9 | 3.42g (19.7 mmol) | 0.73g (2.59 mmol) | 1.3g (3.63 mmol) | 8.12g (131 mmol) | 1.61g (25.92 mmol) | 32.8 mg (0.104 mmol) | 6900 | - |
| Ex. 10 | 2.51g (14.4 mmol) | 1.36g (4.81 mmol) | 1.73g (4.81 mmol) | 7.54g (121.5 mmol) | 1.49g (24.02 mmol) | 30.5 mg (0.096m mol) | 1440 0 | 3 °C |
| Ex. 11 | 3.03g (17.4 mmol) | 0.7g (2.48m mol) | 1.79g (4.97 mmol) | 7.8g (125.6 mmol) | 1.54g (24.85 mmol) | 31.5 mg (0.1 mmol) | 2020 0 | 0 °C |
| Ex. 12 | 2.48g (14.26 mmol) | 1.07g (3.8 mmol) | 2.05g (5.7 mmol) | 7.45g (120 mmol) | 1.47g (23.76 mmol) | 30.1 mg (0.095m mol) | 1820 0 | 5 °C |
| Ex. 13 | 1.5g (8.62 mmol) | 1.46g (5.17 mmol) | 2.79g (7.76 mmol) | 6.76g (108.9 mmol) | 1.33g (21.55 mmol) | 27.3 mg (0.086m mol) | 5100 | 0 °C |
| Ex. 14 | 1.34g (7.71 mmol) | 1.81g (6.43 mmol) | 2.62g (7.28 mmol) | 6.72g (108.2 mmol) | 1.33g (21.42 mmol) | 27.1 mg (0.086m mol) | 5000 | 27 °C |

### Biodegradability test results

The biodegradability test was done according to OECD (1992), Test No. 302B: Inherent Biodegradability: Zahn-Wellens/ EVPA Test, OECD Guidelines for the Testing of Chemicals, Section 3, OECD Publishing, Paris.

To summarize, in this test, a mixture containing the test substance, mineral nutrients and a relatively large amount of activated sludge in aqueous medium is agitated and aerated at 20-25°C in the dark or in diffuse light for up to 28 days. Blank controls, containing activated sludge and mineral nutrients but no test substance, are run in parallel. The biodegradation process is monitored by determination of DOC (Dissolved Organic Carbon) or COD (Chemical Oxygen Demand) in filtered samples taken at daily or other time intervals. The ratio of eliminated DOC (or COD), corrected for the blank, after each time interval, to the initial DOC value is expressed as the percentage biodegradation at the sampling time. The percentage biodegradation is plotted against time to give the biodegradation curve.

The degree of biodegradation attained at the end of the test after 28d, or earlier if complete degradation is attained in less than 28d, is the "inherent biodegradability" of the polymer.

As shown in Table 3 below, the polyester polymers of Examples 1 to 3, 8, 10 and 15 (comprising less than 20 mol% of aromatics) are inherently more biodegradable than those of Examples 4 to 7 (which comprise more than 20 mol% of aromatics).

**Table 3**

| Example N° | % Biodegradation after 28 days |
|---|---|
| 1 | 89 |
| 2 | 80 |
| 3 | 82 |
| 4 | 67 |
| 5 | 66 |
| 6 | 63 |
| 7 | 52 |
| 8 | 92 |
| 10 | 75 |

### Hair straightening performances

### Straightening Test Protocol

### Pretreatment

Natural mixed race Curl Level 3 Hair tresses of 2,7g were used in the studies. All tresses were pretreated with 1 ml of solution of sodium laureth sulfate at 14% in active, to remove any impurities. The tresses were dried at 25C overnight. For each experiment (treatment) 3 tresses were used (i.e. the experiments were made in triplicate).

### Treatment & measurement

The steps of the treatment were as follows:

Step 0: Measure the volume of the tresses before treatment but after the pretreatment step when the hair has been dried.
Step 1: Soak the tresses in tap water at 37°C during 10 min
Step 2: Manually remove water excess and comb the tresses
Step 3: Soak the tresses during 3 min in a 1% w/w polymer aqueous solution
Step 4: Manually remove excess solution and comb the tresses until hair is detangled
Step 5: Dry the tresses using a hair drier for 1min30 on each side. Keep the hair fibers aligned during drying.
Step 6: Apply a flat iron set at 230°C five times, with each pass lasting 5 sec
Step 7: Measure the volume of the treated tresses using the Bolero device (see below). This constitutes T=0.
Step 8: Place all tresses in a climatic chamber set at 20°C and 50%R.H. or 25°C and 80% R.H.
Step 9: Measure the volume of the tresses at T=4, T=24, T=48 and T=72 hours

### Measurement & calculation details:

The measurements of the hair bulk volume and fly away frizz were realized with a Bolero device from Bossa Nova Technologies. These parameters are defined as follows:
- The BULK VOLUME (in cm3), corresponds to the area which shows the highest hair density.
- The FLY AWAY FRIZZ (=FAF in cm3), represents the independent hair which is outside the bulk. A tress is well straightened if there is no fly away frizz.

As an indication of the straightening effect, the following values were calculated:
%Bulk volume reduction = 100 - 100 *( Bulk volume/Initial Bulk volume)
%FAF volume reduction = 100 - 100 *(FAF volume/Initial FAF volume)

In these equations, the initial values refer to the values measured on the tresses before treatment but after the pretreatment described above. This is a necessary step as tresses differ in their initial volumes despite having the same mass, which is attributable to the tress mechanical thermal and humidity history.

The higher the values, the higher is the straightening efficiency.

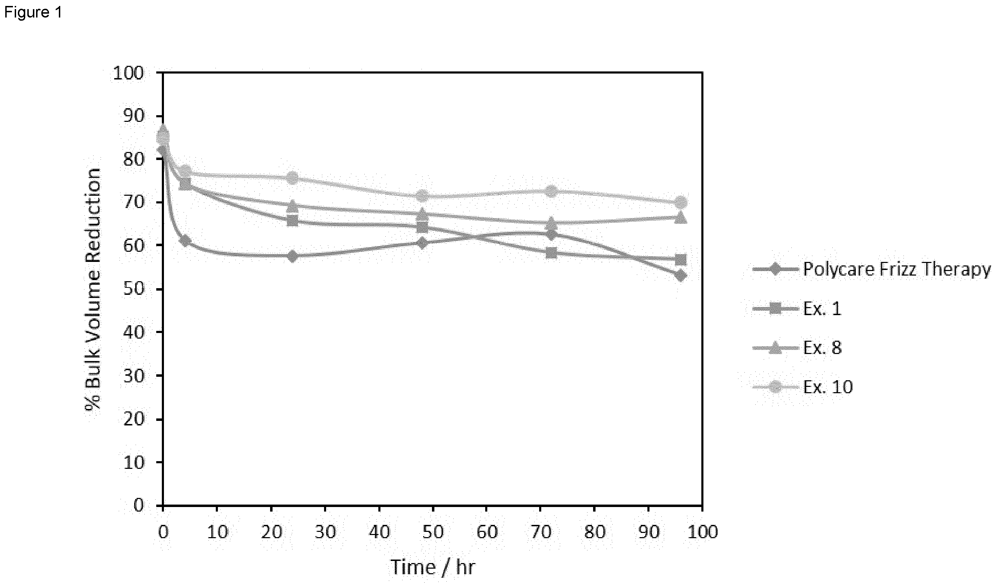

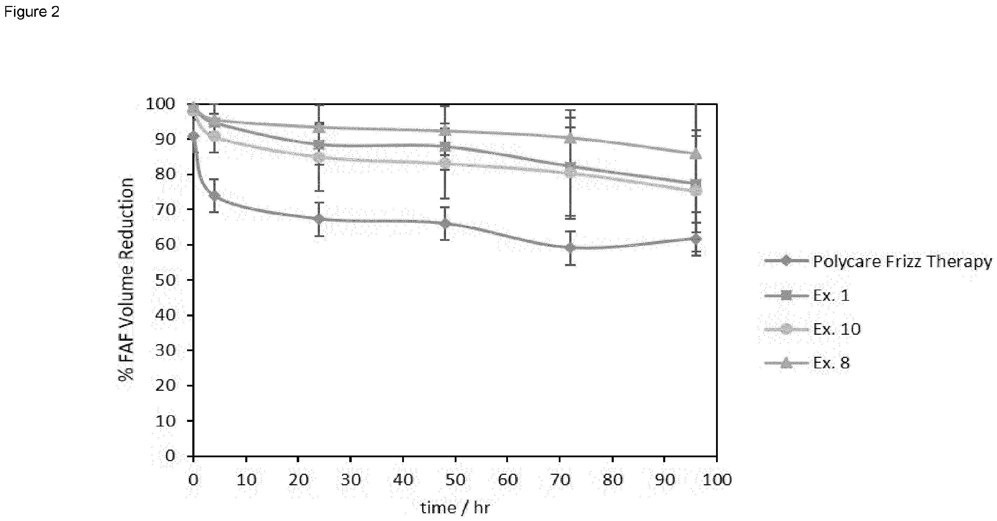

The results obtained with the polyester polymers of Examples 1, 8, 10 and with Polycare^{®} Frizz Therapy (a commercial conditioning agent) are shown in Figures 1 and 2 attached.

## Claims

1. A polyester polymer prepared by polycondensation of at least the following monomers:
(a) an aliphatic/aromatic unsulfonated dicarboxylic acid/ester;
(b) an aliphatic diol;
(c) an aliphatic/aromatic sulfonated dicarboxylic acid/ester;
(d) an aliphatic/aromatic secondary or tertiary amine bearing two carboxylic acid/ester functions, or an aliphatic/aromatic quaternized amine bearing two carboxylic acid/ester functions.

2. The polyester polymer according to claim 1, wherein the aliphatic/aromatic unsulfonated dicarboxylic acid/ester (a) is selected from:
- alkyldicarboxylic acids/esters of formula ROOC-(CH2)n-COOR (I) where n = 2-4 and R is H, a C1 to C8 alkyl or phenyl group;
- cyclopentanedicarboxylic acids/alkyl esters or cyclohexanedicarboxylic acids/alkyl esters, in particular 1,2-cyclopentanedicarboxylic acid, 1,3-cyclopentanedicarboxylic acid, 1,3-cyclohexanedicarboxylic acid or 1,4-cyclohexanedicarboxylic acid and their corresponding alkyl esters where the alkyl group can vary from methyl to octyl or can be a phenyl group;
- terephthalic acid/alkyl esters and isophthalic acid/alkyl esters where the alkyl group can vary from methyl to octyl or can be a phenyl group.

3. The polyester polymer according to claim 2, wherein the aliphatic/aromatic unsulfonated dicarboxylic acid/ester (a) is an aliphatic one, preferably a cyclohexane derivative, in particular 1,4-cyclohexanedicarboxylic acid (CHDA).

4. The polyester polymer according to any one of claims 1 to 3, wherein the aliphatic diol (b) is selected from:
- alkyl diols like ethylene glycol or propylene glycol, diethylene glycol, triethylene glycol; a polyethylene glycol having an ethylene oxide number ranging from ranging from 4 to 75;
- cyclic saturated diols, preferably cyclopentane diols or cyclohexane diols, in particular 1,2-cyclopentane diol, 1,3-cyclopentane diol, 1,3-cyclohexane diol or 1,4-cyclohexane diol.

5. The polyester polymer according to claim 4, wherein the aliphatic diol (b) is an alkyl diol, in particular ethylene glycol (EG).

6. The polyester polymer according to any one of claims 1 to 5, wherein the aliphatic/aromatic sulfonated dicarboxylic acid/ester (c) is selected from:
- isophthalic acid/esters, terephthalic acid/esters and naphthalenedicarboxylic acids/esters, in particular 2-sodiosulfoisophthalic acid/esters, 4-sodiosulfoisophthalic acid/esters, 5-sodiosulfoisophthalic acid/esters, 2-sodiosulfoterephthalic acid/esters, 2,6-dicarboxyl naphthalene-4-sodiosulfonic acid/esters and 2,6-dicarboxyl naphthalene-7-sodiosulfonic acid/esters;
- dialkyl sodium sulfosuccinates.

7. The polyester polymer according to claim 6, wherein the aliphatic/aromatic sulfonated (c) is an aromatic sulfonated dicarboxylic acid/ester, preferably 5-sodiosulfoisophthalic acid or an ester thereof, in particular 5-sodiosulfoisophthalic acid (SSIA).

8. The polyester polymer according to any of claims 1 to 7, wherein monomer (d) is selected from:
- N-methyldiethanolamine, ethylenediamine N,N' diacetic acid/alkyl diesters and iminodiacetic acid/ alkyl diesters, where the alkyl group can vary from methyl to octyl or can be a phenyl group;
- pyridine derivatives like pyridine-2,6-dicarboxylic acid/alkyl diesters, pyridine-2,4-dicarboxylic acid/alkyl diesters, pyridine-3,5-dicarboxylic acid/alkyl diesters, pyridine-2,3-dicarboxylic acid/alkyl diesters and pyridine-2,5-dicarboxylic acid/alkyl diesters; of pyrazine derivatives like pyrazine-2,6-dicarboxylic acid/alkyl diesters, pyrazine-2,3-dicarboxylic acid/alkyl diesters and pyrazine-2,5-dicarboxylic acid/alkyl diesters; of imidazole derivatives like 1H-imidazole-2,5-dicarboxylic acid/alkyl diesters and 1H-imidazole-4,5-dicarboxylic acid/alkyl diesters; and of indole derivatives like 1H-indole-4,6-dicarboxylic acid/alkyl diesters, 1H-indole-2,5-dicarboxylic acid/alkyl diesters, 1H-indole-2,6-dicarboxylic acid/alkyl diesters, 1H-indole-3,5-dicarboxylic acid/alkyl diesters and 1H-indole-2,3-dicarboxylic acid/alkyl diesters, where the alkyl group can vary from methyl to octyl or can be a phenyl group.

9. The polyester polymer according to any of claims 1 to 7, wherein monomer (d) is a quaternized form of an aromatic tertiary amine, preferably of a pyridine derivative, in particular of pyridine-2,6-dicarboxylic acid/esters, more particularly 2,6-bis(methoxycarbonyl)-1-methylpyridin-1-ium trifluoromethanesulfonate.

10. The polyester polymer according to claim 8 or 9, wherein monomer (d) is an aromatic tertiary amine or quaternized amine, preferably pyridine-2,6-dicarboxylic acid (PDA) or 2,6-bis(methoxycarbonyl)-1-methylpyridin-1-ium trifluoromethanesulfonate (quatPDA).

11. The polyester polymer according to any of claims 1 to 10, said polyester being prepared by polycondensation of the following monomers:
(a) 1,4-cyclohexanedicarboxylic acid (CHDA);
(b) ethylene glycol (EG);
(c) 5-sodiosulfoisophthalic acid (SSIA);
(d) pyridine-2,6-dicarboxylic acid (PDA) or 2,6-bis(methoxycarbonyl)-1-methylpyridin-1-ium trifluormethanesulfonate (quatPDA).

12. A polyester polymer comprising the following repeat units: CHDA-EG, SSIA-EG and (quat)PDA-EG.

13. A process for synthesizing a polyester polymer according to any of claims 1 to 11, said process comprising a step of polycondensation of monomers (a), (b), (c) and (d) in the presence of a catalyst, preferably a hydrolysis-stable catalyst, more preferably a chelate of a titanium salt or of zirconium salt derived from ethanol amines, in particular Titanium(IV) (triethanolaminato)isopropoxide.

14. The process according to claim 13, wherein monomers (a), (b), (c) and (d) are first mixed and then reacted by polycondensation by raising temperature and/or reducing pressure.

15. The use of a polyester polymer according to any of claims 1 to 12 or obtained by a process according to claim 13 or 14, in hair care compositions, in particular in hair straightening compositions.

## Patentansprüche

1. Polyesterpolymer, hergestellt durch Polykondensation von mindestens den folgenden Monomeren:
(a) eine aliphatische/aromatische unsulfonierte Dicarbonsäure/einen aliphatischen/aromatischen unsulfonierten Dicarbonsäureester,
(b) ein aliphatisches Diol,
(c) eine aliphatische/aromatische sulfonierte Dicarbonsäure/einen aliphatischen/aromatischen sulfonierten Dicarbonsäureester,
(d) ein aliphatisches/aromatisches sekundäres oder tertiäres Amin, das zwei Carbonsäure/Ester-Funktionen trägt, oder ein aliphatisches/aromatisches quaternisiertes Amin, das zwei Carbonsäure/Ester-Funktionen trägt.

2. Polyesterpolymer nach Anspruch 1, wobei die aliphatisch/aromatische unsulfonierte Dicarbonsäure/der aliphatisch/aromatische unsulfonierte Ester (a) ausgewählt ist aus:
- Alkyldicarbonsäuren/Alkyldicarbonsäureestern der Formel ROOC-(CH2)n-COOR (I), wobei n = 2-4 und R für H, eine C1- bis C8-Alkyl- oder Phenylgruppe steht,
- Cyclopentandicarbonsäuren/ Cyclopentandicarbonsäurealkylestern oder Cyclohexandicarbonsäuren/Cyclohexandicarbonsäurealkyles tern, insbesondere 1,2-Cyclopentandicarbonsäure, 1,3-Cyclopentandicarbonsäure, 1,3-Cyclohexandicarbonsäure oder 1,4-Cyclohexandicarbonsäure und deren entsprechenden Alkylester, wobei die Alkylgruppe von Methyl zu Octyl variieren kann oder eine Phenylgruppe sein kann,
- Terephthalsäure/Terephthalsäurealkylestern und Isophthalsäure/Isophthalsäurealkylestern, wobei die Alkylgruppe von Methyl zu Octyl variieren kann oder eine Phenylgruppe sein kann.

3. Polyesterpolymer nach Anspruch 2, **dadurch gekennzeichnet, dass** die aliphatische/aromatische unsulfonierte Dicarbonsäure/der aliphatische/aromatische unsulfonierten Dicarbonsäureester (a) aliphatisch ist, vorzugsweise ein Cyclohexanderivat, insbesondere 1,4-Cyclohexandicarbonsäure (CHDA).

4. Polyesterpolymer nach einem der Ansprüche 1 bis 3, wobei das aliphatische Diol (b) ausgewählt ist aus:
- Alkyldiole wie Ethylenglykol oder Propylenglykol, Diethylenglykol, Triethylenglykol, einem Polyethylenglykol mit einer Ethylenoxidzahl im Bereich von 4 bis 75,
- cyclischen gesättigten Diolen, vorzugsweise Cyclopentandiolen oder Cyclohexandiolen, insbesondere 1,2-Cyclopentandiol, 1,3-Cyclopentandiol, 1,3-Cyclohexandiol oder 1,4-Cyclohexandiol.

5. Polyesterpolymer nach Anspruch 4, wobei das aliphatische Diol (b) ein Alkyldiol, insbesondere Ethylenglykol (EG), ist.

6. Polyesterpolymer nach einem der Ansprüche 1 bis 5, wobei die aliphatisch/aromatische sulfonierte Dicarbonsäure/der aliphatisch/aromatische sulfonierte Ester (c) ausgewählt ist aus:
- Isophthalsäure/Isophthalsäureestern, Terephthalsäure/Terephthalsäureestern und Naphthalindicarbonsäuren/Naphthalindicarbonsäureestern, insbesondere 2-Sodisulfoisophthalsäure/2-Sodisulfoisophthalsäureestern, 4-Sodisulfoisophthalsäure/2-Sodisulfoisophthalsäureestern, 5-Sodisulfoisophthalsäure/2-Sodisulfoisophthalsäureestern, 2-Sodisulfoterephthalsäure/2-Sodisulfoterephthalsäureestern, 2,6-Dicarboxylnaphthalin-4-sodiosulfonsäure/2,6-Dicarboxylnaphthalin-4-sodisulfonsäureestern und 2,6-Dicarboxylnaphthalin-7-sodiosulfonsäure/2,6-Dicarboxylnaphthalin-7-sodisulfonsäureestern,
- Dialkylnatriumsulfosuccinaten.

7. Polyesterpolymer nach Anspruch 6, wobei das aliphatisch/aromatisch sulfonierte (c) eine aromatische sulfonierte Dicarbonsäure/ein aromatischer sulfonierter Dicarbonsäureester, vorzugsweise 5-Sodiosulfoisophthalsäure oder ein Ester davon, insbesondere 5-Sodiosulfoisophthalsäure (SSIA), ist.

8. Polyesterpolymer nach einem der Ansprüche 1 bis 7, wobei Monomer (d) ausgewählt ist aus:
- N-Methyldiethanolamin, Ethylendiamin-N,N'-diessigsäure/Ethylendiamin-N,N'-diessigsäurealkyldiestern und Iminodiessigsäure/Iminodiessigsäurealkyldiestern, wobei die Alkylgruppe von Methyl zu Octyl variieren kann oder eine Phenylgruppe sein kann,
- Pyridinderivaten wie Pyridin-2,6-dicarbonsäure/Pyridin-2,6-dicarbonsäurealkyldiestern, Pyridin-2,4-dicarbonsäure/Pyridin-2,4-dicarbonsäurealkyldiestern, Pyridin-3,5-dicarbonsäure/alkyldiestern, Pyridin-2,3-dicarbonsäure/Alkyldiester und Pyridin-2,5-dicarbonsäure/Pyridin-2,5-dicarbonsäurealkyldiestern; Pyrazinderivaten wie Pyrazin-2,6-dicarbonsäure/Pyrazin-2,6-dicarbonsäurealkyldiestern, Pyrazin-2,3-dicarbonsäure/Pyrazin-2,3-dicarbonsäurealkyldiester und Pyrazin-2,5-dicarbonsäure/Pyrazin-2,5-dicarbonsäurealkyldiestern; Imidazolderivaten wie 1H-Imidazol-2,5-dicarbonsäure/1H-Imidazol-2,5-dicarbonsäurealkyldiestern und 1H-Imidazol-4,5-dicarbonsäure/1H-Imidazol-4,5-dicarbonsäurealkyldiestern; und Indolderivaten wie 1H-Indol-4,6-dicarbonsäure/1H-Indol-4,6-dicarbonsäurealkyldiestern, 1H-Indol-2,5-dicarbonsäure/1H-Indol-2,5-dicarbonsäurealkyldiestern, 1H-Indol-2,6-dicarbonsäure/1H-Indol-2,6-dicarbonsäurealkyldiestern, 1H-Indol-3,5-dicarbonsäure/1H-Indol-3,5-dicarbonsäurealkyldiestern und 1H-Indol-2,3-dicarbonsäure/1H-Indol-2,3-dicarbonsäurealkyldiestern, wobei die Alkylgruppe von Methyl zu einer Octylgruppe variieren kann oder eine Phenylgruppe sein kann.

9. Polyesterpolymer nach einem der Ansprüche 1 bis 7, wobei Monomer (d) eine quaternisierte Form eines aromatischen tertiären Amins, vorzugsweise eines Pyridinderivats, insbesondere von Pyridin-2,6-dicarbonsäure/Pyridin-2,6-dicarbonsäureestern, insbesondere 2,6-Bis(methoxycarbonyl)-1-methylpyridin-1-iumtrifluormethansulfonat ist.

10. Polyesterpolymer nach Anspruch 8 oder 9, wobei Monomer (d) ein aromatisches tertiäres Amin oder quaternisiertes Amin ist, vorzugsweise Pyridin-2,6-dicarbonsäure (PDA) oder 2,6-Bis(methoxycarbonyl)-1-methylpyridin-1-iumtrifluormethansulfonat (quatPDA).

11. Polyesterpolymer nach einem der Ansprüche 1 bis 10, wobei der Polyester durch Polykondensation der folgenden Monomere hergestellt wird:
(a) 1,4-Cyclohexandicarbonsäure (CHDA),
(b) Ethylenglykol (EG),
(c) 5-Sodiosulfoisophthalsäure (SSIA),
(d) Pyridin-2,6-dicarbonsäure (PDA) oder 2,6-Bis(methoxycarbonyl)-1-methylpyridin-1-iumtrifluormethansulfonat (quatPDA).

12. Polyesterpolymer, welches die folgenden Wiederholungseinheiten umfasst: CHDA-EG, SSIA-EG und (quat) PDA-EG.

13. Verfahren zur Synthese eines Polyesterpolymers nach einem der Ansprüche 1 bis 11, wobei das Verfahren einen Schritt der Polykondensation der Monomere (a), (b), (c) und (d) in Gegenwart eines Katalysators, vorzugsweise eines hydrolysestabilen Katalysators, besonders bevorzugt eines von Ethanolaminen abgeleiteten Chelats eines Titansalzes oder eines Zirkoniumsalzes, insbesondere Titan(IV)-(triethanolaminato)isopropanolat, umfasst.

14. Verfahren nach Anspruch 13, wobei die Monomere (a), (b), (c) und (d) zunächst gemischt und anschließend durch Polykondensation durch Temperaturerhöhung und/oder Drucksenkung umgesetzt werden.

15. Verwendung eines Polyesterpolymers nach einem der Ansprüche 1 bis 12 oder erhalten nach einem Verfahren nach Anspruch 13 oder 14 in Haarpflegemitteln, insbesondere in Haarglättungsmitteln.

## Revendications

1. Polymère de polyester préparé par polycondensation d'au moins les monomères suivants :
(a) un acide/ester dicarboxylique non sulfoné aliphatique/aromatique ;
(b) un diol aliphatique ;
(c) un acide/ester dicarboxylique sulfoné aliphatique/aromatique ;
(d) une amine secondaire ou tertiaire aliphatique/aromatique portant deux fonctions acide/ester carboxylique, ou une amine quaternisée aliphatique/aromatique portant deux fonctions acide/ester carboxylique.

2. Polymère de polyester selon la revendication 1, dans lequel l'acide/ester dicarboxylique non sulfoné aliphatique/aromatique (a) est choisi parmi :
- les acides/esters alkyldicarboxyliques de formule ROOC-(CH2)n-COOR (I) où n = 2-4 et R est H, un groupe alkyle en C1 à C8 ou phényle ;
- les acides/esters d'alkyle cyclopentanedicarboxyliques ou les acides/esters d'alkyle cyclohexanedicarboxyliques, notamment l'acide 1,2-cyclopentanedicarboxylique, l'acide 1,3-cyclopentanedicarboxylique, l'acide 1,3-cyclohexanedicarboxylique ou l'acide 1,4-cyclohexanedicarboxylique et leurs esters d'alkyle correspondants où le groupe alkyle peut varier de méthyle à octyle ou peut être un groupe phényle ;
- l'acide téréphtalique/les esters d'alkyle et l'acide isophtalique/les esters d'alkyle où le groupe alkyle peut varier de méthyle à octyle ou peut être un groupe phényle.

3. Polymère de polyester selon la revendication 2, dans lequel l'acide/ester dicarboxylique aliphatique/aromatique non sulfoné (a) en est un aliphatique, de préférence un dérivé de cyclohexane, en particulier l'acide 1,4-cyclohexanedicarboxylique (CHDA) .

4. Polymère de polyester selon l'une quelconque des revendications 1 à 3, le diol aliphatique (b) étant choisi parmi :
- les alkyldiols, comme l'éthylène glycol ou le propylène glycol, le diéthylène glycol, le triéthylène glycol; un polyéthylène glycol ayant un nombre d'oxyde d'éthylène allant de 4 à 75 ;
- les diols saturés cycliques, de préférence des cyclopentane diols ou des cyclohexane diols, en particulier le 1,2-cyclopentane diol, le 1,3-cyclopentane diol, le 1,3-cyclohexane diol ou le 1,4-cyclohexane diol.

5. Polymère de polyester selon la revendication 4, dans lequel le diol aliphatique (b) est un alkyldiol, en particulier l'éthylèneglycol (EG).

6. Polymère de polyester selon l'une quelconque des revendications 1 à 5, l'acide/ester dicarboxylique sulfoné aliphatique/aromatique (c) étant choisi parmi :
- les acides/esters isophtaliques, les acides/esters téréphtaliques et les acides/esters naphtalènedicarboxyliques, en particulier les acides/esters 2-sodiosulfoisophtaliques, les acides/esters 4-sodiosulfoisophtaliques, les acides/esters 5-sodiosulfoisophtaliques, les acides/esters 2-sodiosulfotéréphtaliques, les acides 2,6-dicarboxylnaphtalène-4-sodiosulfoniques et les acides/esters 2,6-dicarboxylnaphtalène-7-sodiosulfoniques ;
- les sulfosuccinates de dialkyle de sodium.

7. Polymère de polyester selon la revendication 6, dans lequel le (c) sulfoné aliphatique/aromatique est un acide/ester dicarboxylique aromatique sulfoné, de préférence l'acide 5-sodiosulfoisophtalique ou un ester de celui-ci, en particulier l'acide 5-sodiosulfoisophtalique (SSIA).

8. Polymère de polyester selon l'une quelconque des revendications 1 à 7, le monomère (d) étant choisi parmi :
- N-méthyldiéthanolamine, acide/diesters d'alkyle d'acide éthylènediamine N,N' diacétique acide/diesters d'alkyle d'acide iminodiacétique, le groupe alkyle pouvant varier de méthyle à octyle ou pouvant être un groupe phényle ;
- les dérivés de pyridine comme acide/diesters d'alkyle d'acide pyridine-2,6-dicarboxylique, acide/diesters d'alkyle d'acide pyridine-2,4-dicarboxylique, acide/diesters d'alkyle d'acide pyridine-3,5-dicarboxylique, acide/diesters d'alkyle d'acide pyridine-2,3-dicarboxylique et acide/diesters d'alkyle d'acide pyridine-2,5-dicarboxylique ; de dérivés de pyrazine comme acide/diesters d'alkyle d'acide pyrazine-2,6-dicarboxylique, acide/diesters d'alkyle d'acide pyrazine-2,3-dicarboxylique et acide/diesters d'alkyle d'acide pyrazine-2,5-dicarboxylique ; de dérivés d'imidazole comme acide/diesters d'alkyle d'acide 1H-imidazole-2,5-dicarboxylique et acide/diesters d'alkyle d'acide 1H-imidazole-4,5-dicarboxylique ; et de dérivés d'indole comme acide/diesters d'alkyle d'acide 1H-indole-4,6-dicarboxylique, acide/diesters d'alkyle d'acide 1H-indole-2,5-dicarboxylique, acide/diesters d'alkyle d'acide 1H-indole-2,6-dicarboxylique, acide/diesters d'alkyle d'acide 1H-indole-3,5-dicarboxylique et acide/diesters d'alkyle d'acide 1H-indole-2,3-dicarboxylique, où le groupe alkyle peut varier de méthyle à octyle ou peut être un groupe phényle.

9. Polymère de polyester selon l'une quelconque des revendications 1 à 7, dans lequel le monomère (d) est une forme quaternisée d'une amine tertiaire aromatique, de préférence d'un dérivé de pyridine, en particulier d'acide/esters pyridine-2,6-dicarboxylique, plus particulièrement trifluorométhanesulfonate de 2,6-bis(méthoxycarbonyl)-1-méthylpyridin-1-ium.

10. Polymère de polyester selon la revendication 8 ou 9, dans lequel le monomère (d) est une amine tertiaire aromatique ou une amine quaternisée, de préférence acide pyridine-2,6-dicarboxylique (PDA) ou trifluorométhanesulfonate de 2,6-bis(méthoxycarbonyl)-1-méthylpyridin-1-ium (quatPDA).

11. Polymère de polyester selon l'une quelconque des revendications 1 à 10, ledit polyester étant préparé par polycondensation des monomères suivants :
(a) acide 1,4-cyclohexanedicarboxylique (CHDA) ;
(b) éthylèneglycol (EG) ;
(c) acide 5-sodiosulfoisophtalique (SSIA) ;
(d) acide pyridine-2,6-dicarboxylique (PDA) ou trifluorméthanesulfonate de 2,6-bis(méthoxycarbonyl)-1-méthylpyridin-1-ium (quatPDA).

12. Polymère de polyester comprenant les motifs répétitifs suivants : CHDA-EG, SSIA-EG et (quat)PDA-EG.

13. Procédé de synthèse d'un polymère de polyester selon l'une quelconque des revendications 1 à 11, ledit procédé comprenant une étape de polycondensation de monomères (a), (b), (c) et (d) en présence d'un catalyseur, de préférence un catalyseur stable à l'hydrolyse, plus préférablement un chélate d'un sel de titane ou de sel de zirconium dérivé d'éthanolamines, notamment (triéthanolaminato)isopropoxyde de titane(IV).

14. Procédé selon la revendication 13, dans lequel les monomères (a), (b), (c) et (d) sont d'abord mélangés et ensuite mis à réagir par polycondensation en élevant la température et/ou en réduisant la pression.

15. Utilisation d'un polymère de polyester selon l'une quelconque des revendications 1 à 12 ou obtenu par un procédé selon la revendication 13 ou 14, dans des compositions de soin capillaire, en particulier dans des compositions de lissage capillaire.
